# EUROPÄISCHE PATENTANMELDUNG

(11) **EP 2 031 531 A2**
(43) Veröffentlichungstag der Anmeldung: **04.03.2009**
(21) Anmeldenummer: 08151038.0
(22) Anmeldetag: 04.02.2008
(51) Int. Cl.: G06F 19/00

(54) **Integriertes medizintechnisches Anzeigesystem**

(30) Priorität: 20.07.2007 EP 07014276
(71) Anmelder: BrainLAB AG, 85622 Feldkirchen (DE)
(72) Erfinder: Steinle, Wolfgang, 80337 München (DE); Frielinghaus, Nils, 85551 Heimstetten (DE); Hamilton, Christoffer, 81371 München (DE)
(74) Vertreter: Schwabe - Sandmair - Marx

(57) **Zusammenfassung**

Die Erfindung betrifft ein medizintechnisches Anzeigesystem mit einer Bildanzeigeeinheit (1), die zur Darstellung von Bilddaten medizinischen oder medizintechnischen Ursprungs angepasst ist, und mit einer systemintegral zur Bildanzeigeeinheit (1) vorgesehenen Zusatzeinrichtung (4, 5, 7, 8, 9, 10, 11, 15, 17, 18), welche die medizintechnische Funktion der Bildanzeigeeinheit (1) unterstützt.

## Beschreibung

Die Erfindung betrifft ein integriertes medizintechnisches Anzeigesystem, das eine Bildanzeigeeinheit aufweist, die zur Darstellung von Bilddaten medizinischen oder medizintechnischen Ursprungs angepasst ist.

Medizintechnisches medizinisches Bildmaterial, das mit Hilfe medizintechnischer Bildgebungsverfahren erzeugt wird, wie z.B. mit Computertomographie, Kernspintomographie oder Röntgengeräten (zweidimensional/dreidimensional), wird in immer größerem Umfang als digitales Bildmaterial bzw. als digitaler Bilddatensatz gespeichert. Hierfür verwendete Systeme tragen die englische Bezeichnung "Picture Archiving and Communication System (PACS)". Die primäre Betrachtung bzw. Auswertung solcher digitalen Bilder beschränkt sich derzeit auf Radiologen, die in dafür vorgesehenen Betrachtungsräumen mit hochauflösenden Monitoren hoher Leuchtkraft arbeiten, wodurch in Krankenhäusern Einsparungen bezüglich des verwendeten Filmmaterials erzielt werden können.

Außerhalb der Radiologie schreitet der Übergang zur filmlosen Bildbetrachtung langsamer fort. Es werden beispielsweise Bilder, die in der Radiologie filmlos betrachtet werden, auf Film ausgedruckt, um einer sekundären Verwendung in anderen Abteilungen zugänglich gemacht zu werden. Dies mag einerseits daran liegen, dass PACS-Computerprogramme sehr auf Radiologen abgestimmt sind; andererseits ist ihre Bedienung oftmals kompliziert. Hinzu kommt noch, dass viele Ärzte daran gewöhnt sind, mit einem von hinten beleuchteten Schaukasten zu arbeiten, der auch "Lightbox" genannt wird.

Es sind Anstrengungen unternommen worden, digitales Bildmaterial für die sekundäre Verwendung außerhalb der Radiologie besser zugänglich zu machen, beispielsweise durch Großbildmonitore in Operationssälen, die durch kabellose Tastaturen oder Mäuse bedient werden können. Auch einfache Touchscreen-Bedienungen werden verwendet, oder es werden separate Kameras bereitgestellt, welche Steuerungseingaben von Ärzten oder Bedienungspersonal erkennen können. Ein Anzeigesystem für medizinische Bilder, das in der Art eines Schaukastens bzw. einer Lightbox aufgebaut ist, ist aus der US-2002/0039084 A1 bekannt.

Andererseits werden Operationssäle in letzter Zeit zunehmend mit technischen Geräten ausgestattet, um den behandelnden Ärzten jedwede mögliche technische Unterstützung zu gewährleisten. Diese "Technisierung" von Operationsräumen resultiert in einem hohen Platzbedarf und darin, dass das Behandlungspersonal die Bedienung vieler einzelner Geräte erlernen muss.

Es ist die Aufgabe der vorliegenden Erfindung, die oben aufgezeigten Probleme zumindest teilweise zu überwinden, insbesondere ein einfaches bzw. einfach bedienbares OP-Setup zu schaffen, das eine weitgehende technische Unterstützung bereitstellt.

Diese Aufgabe wird durch ein integriertes medizintechnisches Anzeigesystem gemäß dem Anspruch 1 gelöst. Die Unteransprüche definieren bevorzugte Ausführungsformen der Erfindung.

Gemäß der vorliegenden Erfindung umfasst das medizintechnische Anzeigesystem die schon oben genannte Bildanzeigeeinheit, die eine medizintechnische Lightbox oder digitale Lightbox (digitaler Schaukasten) bilden kann. Außerdem umfasst das System mindestens eine systemintegral zur Bildanzeigeeinheit vorgesehene Zusatzeinrichtung, welche die medizintechnische Funktion der Bildanzeigeeinheit unterstützt. Mit anderen Worten fasst das erfindungsgemäße System die Bildanzeigeeinheit in integrierter Weise mit einem weiteren Gerät zusammen, welches im Operationssaal gebraucht wird oder dessen Gebrauch Vorteile für das Behandlungspersonal bietet. Die Zusatzeinrichtung kann die Funktion der Bildanzeigeeinrichtung selbst ergänzen, ermöglichen oder wiederherstellen. Weil solche Bildanzeigeeinheiten ohnehin mehr und mehr in technisch hochwertig ausgestatteten Operationssälen vorhanden sind, eignen sie sich hervorragend als Basis- oder Grundelement der erfindungsgemäßen Integration.

Es ist möglich, einen Adapter an der Bildanzeigeeinheit vorzusehen, der zur Befestigung der Zusatzeinrichtung und/oder zum Datentausch zwischen einer Datenverarbeitungseinheit der Bildanzeigeeinheit und der Zusatzeinrichtung dient. Hier zu bemerken, dass viele in Operationssälen verwendete Geräte selbst Daten empfangen, aufnehmen oder weiterleiten bzw. abgeben, und digitale Lightboxen verarbeiten solche Daten, speziell Bilddaten aber auch andere Daten, wie z.B. Daten von Anästhesiegeräten. Gerade in diesem Bereich ist der digitale Datentausch zwischen der Bildanzeigevorrichtung und der Zusatzeinrichtung von Vorteil, insbesondere weil solche Daten direkt angezeigt werden können, um das Behandlungspersonal zu informieren. Natürlich weist ein hierzu fähiges Bildanzeigegerät eine Datenverarbeitungseinheit (mit Speicher und Datenleitung) auf, und wenn im Weiteren von Vorgängen die Rede ist, die eine Datenverarbeitung erfordern, wird diese durch eine solche Datenverarbeitungseinheit vorgenommen. Grundsätzlich kann natürlich eine solche Datenverarbeitung auch extern durch ein separates, angeschlossenes Gerät erfolgen.

Die Zusatzeinrichtung kann eine Einrichtung zur Eingabe oder Ausgabe von Daten in die bzw. aus der Bildanzeigeeinheit sein. Ein Beispiel für eine solche Zusatzeinrichtung ist eine Einrichtung zum Einlesen oder Ausgeben von Hardware-Bildmaterial, insbesondere einen Scanner und/oder ein Drucker, speziell ein Röntgenbildscanner oder Drucker.

Gemäß einer Ausführungsvariante der Erfindung umfasst die Zusatzeinrichtung eine Einrichtung zum Abtasten von dreidimensionalen Objekten und zur Eingabe der Objektdaten in die Bildanzeigeeinheit, insbesondere einen 3D-Scanner für medizintechnische, speziell orthopädische Musterstücke oder Templates.

Die Zusatzeinrichtung kann eine Einrichtung zum Auslösen, Steuern oder Regeln einer Funktion der Bildanzeigeeinheit umfassen, insbesondere eine Sprachsteuerung oder einen virtuellen, projizierten Hand- oder Fußschalter.

Es besteht die Möglichkeit, als Zusatzeinrichtung eine Einrichtung zur Anbringung oder Aufnahme und zur Adaption einer externen Datenverarbeitungseinheit vorzusehen, insbesondere eines tragbaren Computers oder eines Tablet-PCs. Gemäß einer Ausführungsform der Erfindung umfasst die Zusatzeinrichtung eine Einrichtung zur Überwachung des Raumes vor der Bildanzeigeeinheit, insbesondere zur Detektion von Erkennungsmerkmalen, speziell einen RFID-Scanner zur Erkennung von RFID-Tags an Patienten oder medizintechnischen Geräten bzw. Instrumenten.

Gemäß einer möglichen Ausgestaltung hat die Zusatzeinrichtung eine Einrichtung zur visuellen Überprüfung der Anzeigeeinheit selbst, insbesondere zur Überprüfung von Anzeigefläche oder Bildschirm, und diese Einrichtung kann eine auf die Anzeigefläche oder den Bildschirm gerichtete Kamera sein.

Gemäß einer weiteren Ausführung des erfindungsgemäßen Systems umfasst die Zusatzeinrichtung eine Einrichtung zur Erfassung des äußeren Beleuchtungszustandes, entweder direkt durch Messung oder indirekt durch Datenempfang, wobei die Anzeigefläche in einen hell scheinenden Modus oder Notlichtmodus geschaltet werden kann, wenn die äußere Beleuchtung ausfällt oder unzureichend ist. Mit dem Begriff "äußere Beleuchtung" ist hier der Beleuchtungszustand in dem Raum gemeint, in dem sich das System befindet, also beispielsweise im Operationssaal.

Die Zusatzeinrichtung kann eine Einrichtung zur Erfassung eines Benutzer-Blickes umfassen, insbesondere eine Kamera und eine Software zur Erkennung, ob ein Benutzer der Anzeige sein Gesicht zuwendet.

Bei einer weiteren Ausführungsvariante weist die Zusatzeinrichtung eine Einrichtung zum Drehen der Anzeigeeinheit in der Ebene der Anzeigefläche auf, insbesondere zum Verdrehen um 90° in Abhängigkeit vom Format der dargestellten Bilddaten.

Die Erfindung verwendet also insbesondere die zentrale Bilddarstellungseinheit, um Funktionen oder Wartungsaufgaben zu integrieren, die derzeit manuell, mit separaten zusätzlichen Geräten oder überhaupt nicht durchgeführt werden. Sie stellt dem Benutzer in zentraler Weise eine konsistente Schnittstelle zur Verfügung, um eine Vielzahl von Aufgaben zu erledigen, wobei speziell die Qualitätssicherung, der Energieverbrauch und die Ergonomie verbessert werden, weil ein leichter und intuitiver Zugang zu wichtigen Funktionen zur Verfügung gestellt wird.

Die Erfindung wird im Weiteren anhand verschiedener Ausführungsformen und unter Bezugnahme auf die beiliegenden Zeichnungen näher erläutert. Sie kann alle hierin beschriebenen Merkmale einzeln sowie in jedweder sinnvollen Kombination umfassen. Wenn hierin auf einzelne Dokumente Bezug genommen wird, so soll den Inhalt betreffend die jeweilige technische Ausführungsform als durch Bezugnahme hier aufgenommen gelten. In den Zeichnungen, die erfindungsgemäße Ausführungsformen darstellen, zeigen:
- Figur 1: ein Anzeigesystem mit Röntgenbildscannner;
- Figur 2: ein Anzeigesystem mit Röntgenbilddrucker;
- Figur 3: ein Anzeigesystem mit 3D-Scanner;
- Figur 4: ein Anzeigesystem mit Sprachsteuerung;
- Figur 5: ein Anzeigesystem mit Kamera;
- Figur 6: ein Anzeigesystem, das als Notlicht arbeitet;
- Figur 7: ein Anzeigesystem mit RFID-Integration;
- Figur 8: ein Anzeigesystem mit Tablet-PC-Integration;
- Figur 9: ein Anzeigesystem mit Bildschirmüberwachung;
- Figur 10: ein Anzeigesystem mit Drehautomatik; und
- Figur 11: ein Anzeigesystem mit projiziertem Fußschalter.

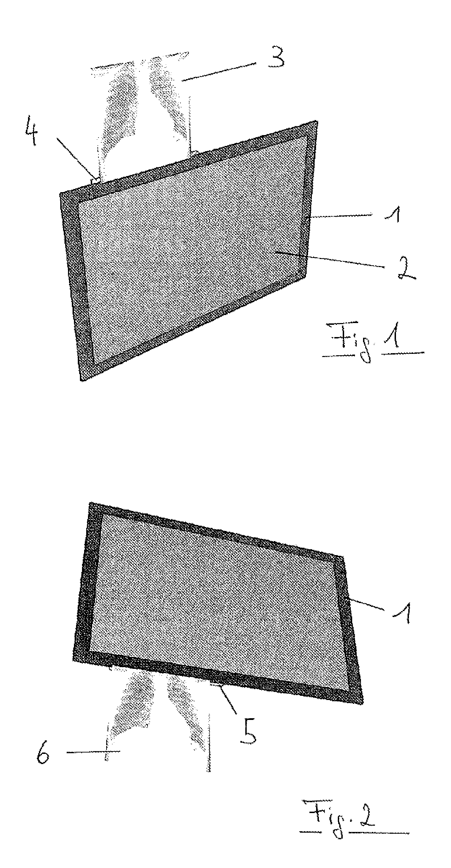

In der Figur 1 und in allen weiteren Figuren deutet das Bezugszeichen 1 auf eine Bildanzeigevorrichtung, hier eine digitale Lightbox, die beispielsweise durch Touch-Screen-Steuerung bedienbar ist. Wo es verwendet wird, zeigt das Bezugszeichen 2 auf die Anzeigefläche bzw. den Bildschirm. Grundsätzlich kann die Bildanzeigeeinheit 1 medizintechnische Bilder, die in digitaler Form bereitgestellt werden, verarbeiten und anzeigen und damit eine visuelle Unterstützung für das Behandlungspersonal bereitstellen.

In Figur 1 ist gezeigt, wie mit der Anzeigeeinheit 1 ein Bildscanner, nämlich ein Röntgenbildscanner 4 integral bereitgestellt wird. Der Röntgenbildscanner 4 macht es möglich, in einfacher Weise ein medizinisches Bild, hier eine Röntgenbildfolie 3, zu digitalisieren und anzuzeigen und/oder dem Krankenhaus-Bildserver-System hinzuzufügen, indem der entsprechende Patientendatensatz im Krankenhaus-Serversystem gesucht und das Bild dann zugeordnet wird. Während des Scan-Prozesses kann außerdem der Inhalt des Röntgenfilmes gleichzeitig auf dem Schirm dargestellt werden, was den Eindruck erweckt, dass sich der Film in den Bildschirm bzw. die Anzeigefläche hineinbewegt.

Die Figur 2 zeigt ein Anzeigesystem mit einem integrierten Drucker 5 am unteren Rand der Bildanzeigeeinheit 1. Mit dem Drucker 5 kann der Benutzer jedwedes Bild ausdrucken, dass er auf dem integrierten System angesehen hat oder das im integrierten System gespeichert ist. Die erzeugten Bilder können dem Patienten mitgegeben werden oder, wenn sie wie hier Röntgenbilder 6 sind, auf herkömmlichen Schaukästen angesehen werden. Um eine solche Röntgenfolie auszudrucken, wird der Touch-Screen benutzt, um beispielsweise ein Bild nach unten zu schieben. Der Ausdruck wird dann simultan erzeugt und kommt unten aus dem Drucker heraus. Dies macht den Eindruck, als würde das digitale, auf dem Schirm dargestellte Bild wie eine Hardware über den Schirm geschoben und dann ausgegeben.

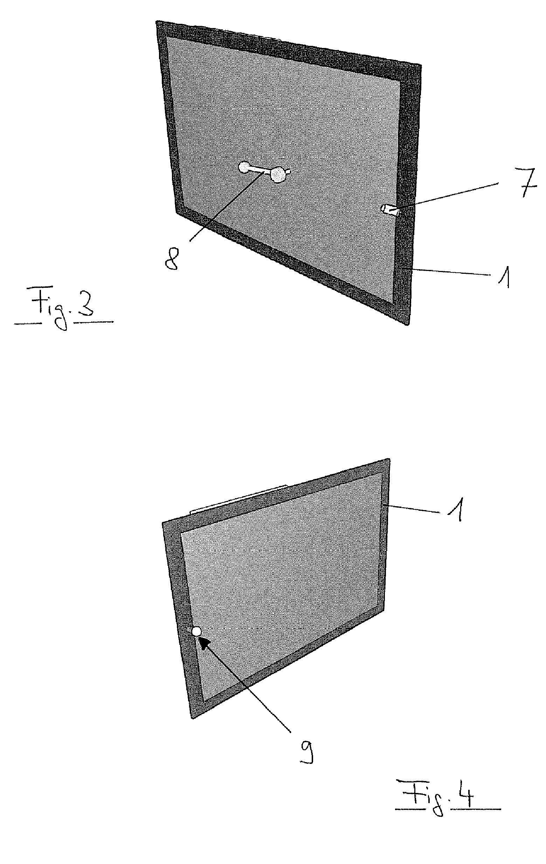

Die Figur 3 zeigt eine Ausführungsform, bei der eine Scanner-Kamera 7 an der Anzeigeeinheit 1 angeordnet ist, mit der die Abmessungen von Objekten bestimmt werden können wie z.B. des dargestellten Objektes 8, das ein medizinisches bzw. orthopädisches Template sein kann. In der US 5,988,862 wird beispielsweise beschrieben, wie die Abmessungen eines dreidimensionalen Objektes durch Scannen bestimmt werden können und wie dann das Objekt als dreidimensionales Datenobjekt in ein CAD-Programm übernommen werden kann. Die hergestellten Modelle können erfindungsgemäß auch modifiziert und in Bezug auf medizinische Bilder und/oder Objekte verschoben werden, indem man sie auf dem Touch-Screen der Bildanzeigeeinheit nach dem Einscannen markiert und manipuliert.

Die Figur 4 zeigt eine Anzeigeeinheit 1 mit einem daran angeordneten Mikrofon 9, durch welches dem System bzw. einem integriertem System per Sprache Anweisungen erteilt werden können. Hierdurch wird eine berührungslose Steuerung und Manipulation ermöglicht, was die einfache Verwendung durch schon sterilisierte Chirurgen möglicht macht.

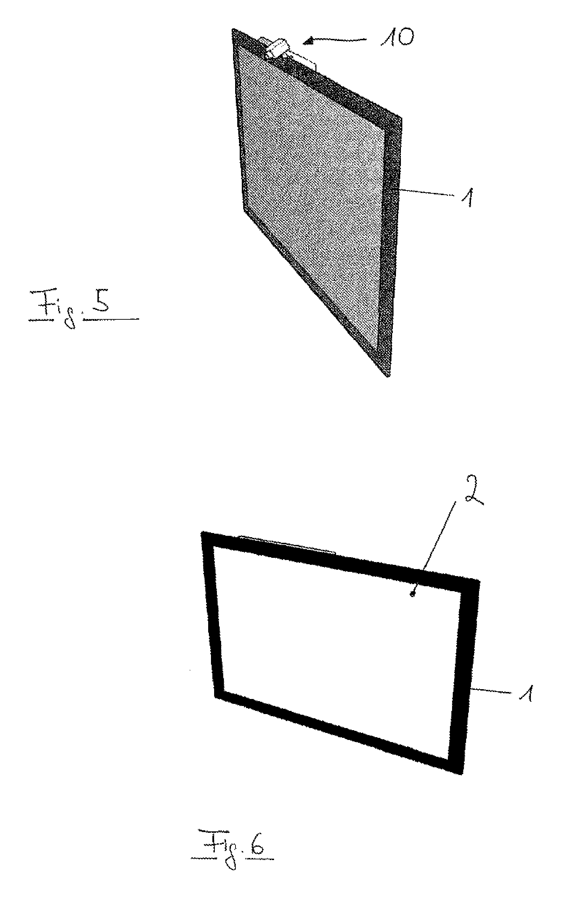

Die Figur 5 zeigt ein erfindungsgemäßes System, das mit einer integrierten Kamera 10 ausgestattet ist. Diese Kamera kann vielfältige Bilddaten aufnehmen und zur Verwendung mit dem System bereitstellen, ein Einsatzfall ist beispielsweise die Erfassung von Benutzer-Blicken. Es gibt verschiedene Techniken, bei denen Helme verwendet werden, um den Blickwinkel von Benutzern festzustellen, und eine davon ist beispielsweise in der US 6,433,759 aufgezeigt. Dabei wird festgestellt, auf welche Teile des Bildschirms ein Benutzer gerade blickt. Obwohl natürlich grundsätzlich eine solche Integration erfindungsgemäß möglich ist, wird vor allen Dingen eine Ausführungsform ins Auge zu fassen sein, bei der die Kamera 10 angeordnet und mit einer Software verbunden wird, welche feststellt, ob überhaupt ein Verwender auf die Anzeigeeinheit 1 blickt. Die Kamera und das Computerprogramm verfolgen dabei alle Personen in der Umgebung des Systems. Wenn dann erfasst wird, dass einer der Verwender auf die Anzeigeeinheit 1 blickt (z.B. wenn ein Gesicht von vorne oder seitlich vorne erkannt wird) wird die Beleuchtung verstärkt, die Anzeige erst eingeschaltet oder eine andere Ansicht gezeigt, die eine bessere Betrachtung erlaubt. Dies resultiert in einer erhöhten Bedienerfreundlichkeit und einer längeren Lebensdauer für die Anzeige.

In der Ausführungsform der Figur 6 wird das integrierte System als Notlichtsystem verwendet. Wenn über einen (hier nicht dargestellten) Sensor an der Einheit 1 oder anderswo im Raum festgestellt wird, dass die Beleuchtung zu dunkel wird, oder wenn ein Stromausfall herrscht, kann das erfindungsgemäße System als Notlicht dienen. Hierzu würde man es mit einer unterbrechungsfreien Stromversorgung (USV) ausstatten, und das helle Anzeigenlicht würde es dann dem Behandlungspersonal im Operationssaal gestatten kritische Aufgaben zu Ende zu bringen.

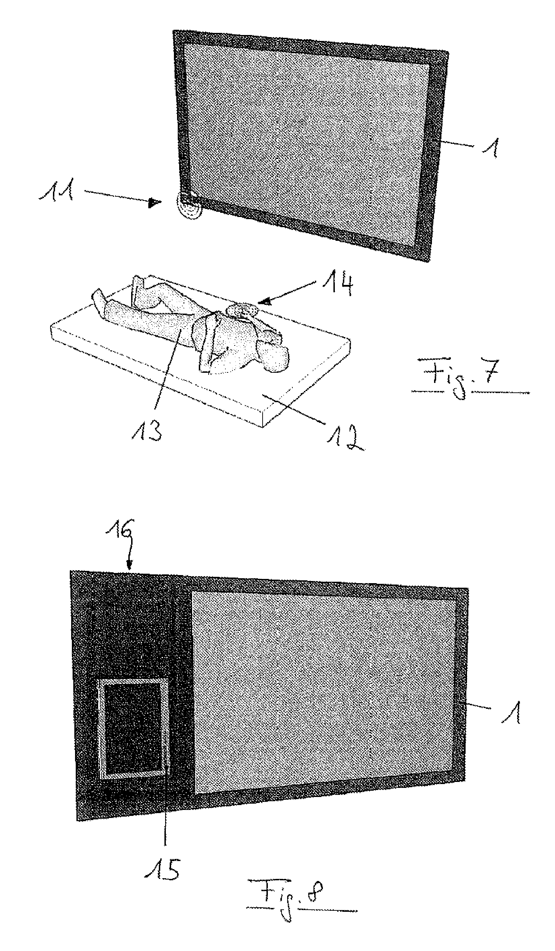

Die Figur 7 stellt eine Ausführungsvariante dar, bei eine die RFID-Technik integriert wird. Die Verbreitung von RFID-Tags in Krankenhausumgebungen macht es möglich, alle Patienten mit RFID-Tags bzw. -sendern auszustatten. In der Figur 7 hat der Patient 13, der auf der Liege 12 liegt, beispielsweise den RFID-Tag 14. An der Anzeigeeinheit 1 ist ein RFID-Scanner 11 integriert vorgesehen, und dieser Scanner scannt aktiv die Umgebung des Systems auf das Vorhandensein von Patienten. Die Bilddaten solcher aufgefundenen Patienten werden dann beispielsweise automatisch aus dem Krankenhausserver geholt und lokal gespeichert. Dadurch wird einerseits die Patientenidentifikation und Auswahl im Softwaresystem vereinfacht und andererseits wird der Prozess für das Laden der Patientendaten beschleunigt.

Ein System, bei dem eine externe Datenverarbeitungseinheit, hier ein Tablet-PC 15 mit der Anzeigeneinheit 1 integriert wird, ist in Figur 8 zu sehen. Im Panelbereich 16 der Einheit 1 ist eine Adapter-Aussparung (docking station) vorgesehen, in die beispielsweise der Tablet-PC eingesetzt werden kann. In einem solchen Fall umfasst dann das integrierte System mehrere Bildanzeigemonitore, und der Tablet-PC 15 und seine Touch-Screen-Oberfläche kann Steuerungsfunktionen für die Bildanzeige bereitstellen. Wenn der Tablet-PC an dem System angebracht ist, arbeitet sein Touch-Screen-Monitor als integraler Teil des Systems; er kann aber auch aus der Einheit 1 entnommen und als separate Einheit verwendet werden. Wenn man eine Synchronisation der Daten bzw. deren Austausch sicherstellt, lassen sich dann die Bilddaten auf dem Tablet-PC auch an einer entfernten Stelle einsehen bzw. manipulieren.

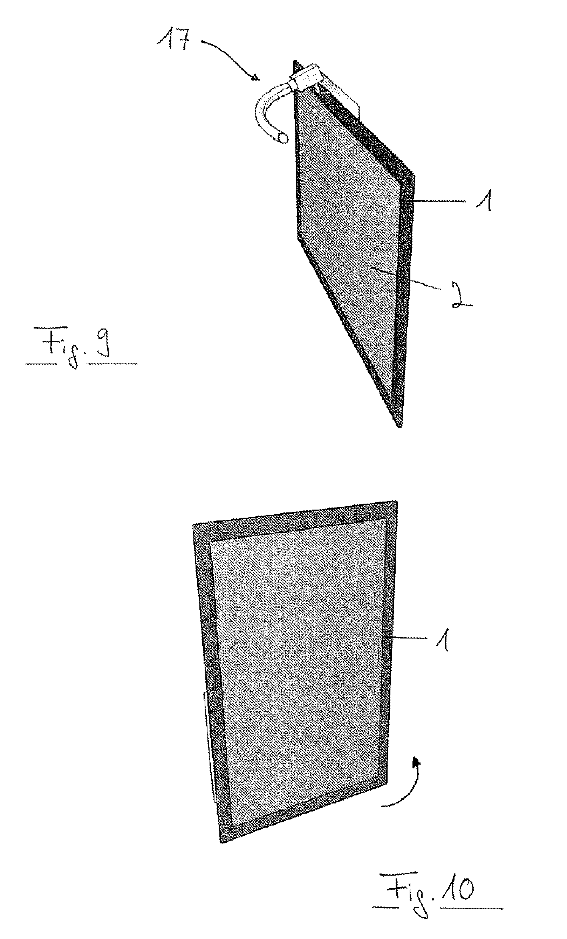

Die in Figur 9 dargestellte Ausführungsform zeigt eine Bildanzeigeeinheit mit integrierter Kamera und einem beweglichen, verlängerten Objektiv oder Lichtsensor. Ein Problem bei Touch-Screen-Monitorsystemen liegt darin, dass Fingerabdrücke auf der Oberfläche die Bildausgabe stören können. Die Ausführungsform der Figur 7 gestattet eine automatische Qualitätskontrolle für die Anzeige, wobei die Kamera 17 Bilder auf der Anzeige 2 aufnimmt. Die Kamerabilder werden dann mit den Bilddaten verglichen, die auf die Anzeige geliefert werden, und wenn Unterschiede vorhanden sind, werden diese Abdrücken oder anderen Bildstörungen oder Monitordefekten (Pixelfehler) zugeschrieben. Die Displays können mechanisch gereinigt werden, wie dies z.B. in der US 6,934,590 beschrieben ist, oder das integrierte System kann eine Reinigung (Reinigungspersonal) oder Wartung anfordern.

Die Figur 10 zeigt eine Ausführungsform mit einer drehbaren Bildanzeigeeinheit 1 (siehe Pfeil). Die Bildanzeigeeinheit 1 wird durch eine bekannte Schwenkeinrichtung in der Anzeigeebene gedreht, wobei die Dreheinrichtung Motoren und Scharniere aufweisen kann, hier aber nicht dargestellt ist. Monitore sind im Allgemeinen rechteckig und haben Seitenverhältnisse von 4:3 oder 16:9. Einige Monitore können um 90° gedreht werden und dabei automatisch die Anzeige ändern. Eine solche Funktion wird nun in entgegengesetzter Weise erfindungsgemäß integriert. Beispielsweise sind CT-Scans mit mehreren Schnittbildansichten meistens Bilder, die im Querformat angezeigt werden, und das erfindungsgemäße System dreht den Monitor in die Querformat-Richtung, wenn solche Bilder dargestellt werden. Andererseits dreht das System die Anzeigeeinheit 1 in das Hochformat, wenn Bilder angezeigt werden, die sich in dieser Darstellung besser betrachten lassen, also beispielsweise Röntgenbilder von Wirbelsäulen.

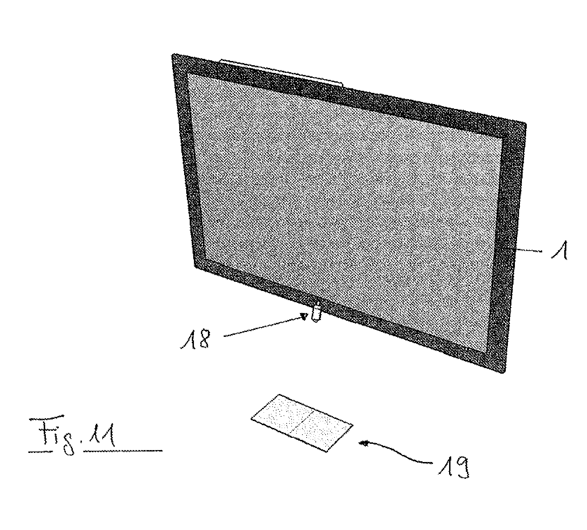

Die Figur 11 zeigt schließlich eine Anzeigeeinrichtung 1 mit einer Projektionsvorrichtung 18, die ein virtuelles Pedal auf den Boden projiziert, das mit dem Bezugszeichen 19 angedeutet ist. Die Haupt-Verwenderschnittstelle im integrierten System wird meist der Multi-Touch-Screen sein. Für einige Tätigkeiten (z.B. Wechsel zum nächsten oder vorherigen Schichtbild) ist es hilfreich, wenn man "Abkürzungen" schafft, bei denen der Verwender nicht unbedingt an das System (Bildschirm) herantreten muss. Fußpedale werden derzeit schon bei mehreren medizinischen Vorrichtungen verwendet. Gemäß der Erfindung wird der Projektor oder Licht-Emitter 18 eine bestimmte Stelle auf den Boden beleuchten und dabei ein oder mehrere Fußschalter virtuell zur Verfügung stellen. Jedes Pedal kann gemäß der derzeitigen Anwendung und seiner Funktion gelabelt werden, und ein optischer Sensor erfasst, ob der Verwender auf einem der virtuellen Fußschalter tritt und überträgt die Information an die derzeit verwendete Software-Anwendung.

## Patentansprüche

1. Medizintechnisches Anzeigesystem mit einer Bildanzeigeeinheit (1), die zur Darstellung von Bilddaten medizinischen oder medizintechnischen Ursprungs angepasst ist, und mit einer systemintegral zur Bildanzeigeeinheit (1) vorgesehenen Zusatzeinrichtung (4, 5, 7, 8, 9, 10, 11, 15, 17, 18), welche die medizintechnische Funktion der Bildanzeigeeinheit (1) unterstützt.

2. System nach Anspruch 1, **dadurch gekennzeichnet, dass** ein Adapter an der Bildanzeigeeinheit (1) vorgesehen ist, der zur Befestigung der Zusatzeinrichtung und/oder zum Datentausch zwischen einer Datenverarbeitungseinheit der Bildanzeigeeinheit (1) und der Zusatzeinrichtung dient.

3. System nach Anspruch 1 oder 2, **dadurch gekennzeichnet, dass** die Zusatzeinrichtung eine Einrichtung zur Eingabe oder Ausgabe von Daten in die bzw. aus der Bildanzeigeeinheit ist.

4. System nach einem der Ansprüche 1 bis 3, **dadurch gekennzeichnet, dass** die Zusatzeinrichtung eine Einrichtung zum Einlesen oder Ausgeben von Hardware-Bildmaterial (3, 6) umfasst, insbesondere einen Scanner (4) und/oder einen Drucker (5), speziell einen Röntgenbildscanner oder -drucker.

5. System nach einem der Ansprüche 1 bis 4, **dadurch gekennzeichnet, dass** die Zusatzeinrichtung eine Einrichtung zum Abtasten von dreidimensionalen Objekten (8) und zur Eingabe der Objektdaten in die Bildanzeigeeinheit (1) umfasst, insbesondere einen 3D-Scanner (7) für medizintechnische, speziell orthopädische Musterstücke (8) oder Templates.

6. System nach einem der Ansprüche 1 bis 5, **dadurch gekennzeichnet, dass** die Zusatzeinrichtung eine Einrichtung zum Auslösen, Steuern oder Regeln einer Funktion der Bildanzeigeeinheit umfasst, insbesondere eine Sprachsteuerung (9) oder einen virtuellen, projizierten Hand- oder Fußschalter (19).

7. System nach einem der Ansprüche 1 bis 6, **dadurch gekennzeichnet, dass** die Zusatzeinrichtung eine Einrichtung zur Anbringung oder Aufnahme und zur Adaption einer externen Datenverarbeitungseinheit, insbesondere eines tragbaren Computers oder eines Tablet-PC (15) umfasst.

8. System nach einem der Ansprüche 1 bis 4, **dadurch gekennzeichnet, dass** die Zusatzeinrichtung eine Einrichtung zur Überwachung des Raumes vor der Bildanzeigeeinheit, insbesondere zur Detektion von Erkennungsmerkmalen umfasst, speziell einen RFID-Scanner (11) zur Erkennung von RFID-Tags (14) an Patienten oder medizintechnischen Geräten bzw. Instrumenten.

9. System nach einem der Ansprüche 1 bis 8, **dadurch gekennzeichnet, dass** die Zusatzeinrichtung eine Einrichtung zur visuellen Überprüfung der Anzeigeeinheit (1) selbst, insbesondere zur Überprüfung von deren Anzeigefläche (2) oder Bildschirm, umfasst, speziell eine auf die Anzeigefläche oder den Bildschirm gerichtete Kamera (10).

10. System nach einem der Ansprüche 1 bis 9, **dadurch gekennzeichnet, dass** die Zusatzeinrichtung eine Einrichtung zur Erfassung des äußeren Beleuchtungszustandes umfasst, entweder direkt durch Messung oder indirekt durch Datenempfang, wobei die Anzeigefläche (2) in einen hell scheinenden Modus oder Notlichtmodus geschaltet werden kann, wenn die äußere Beleuchtung ausfällt oder unzureichend ist.

11. System nach einem der Ansprüche 1 bis 10, **dadurch gekennzeichnet, dass** die Zusatzeinrichtung eine Einrichtung zur Erfassung eines Benutzer-Blickes umfasst, insbesondere eine Kamera (10) und eine Software zur Erkennung, ob ein Benutzer der Anzeige sein Gesichts zuwendet.

12. System nach einem der Ansprüche 1 bis 11, **dadurch gekennzeichnet, dass** die Zusatzeinrichtung eine Einrichtung zum Drehen der Anzeigeeinheit (1) in der Ebene der Anzeigefläche umfasst, insbesondere zum Verdrehen um 90° in Abhängigkeit vom Format der dargestellten Bilddaten.

13. System nach einem der Ansprüche 1 bis 12, **dadurch gekennzeichnet, dass** die Bildanzeigeeinheit (1) eine medizintechnische Lightbox, insbesondere eine digitale Lightbox ist.
